# EUROPÄISCHE PATENTSCHRIFT

(11) **EP 0 883 393 B1**
(45) Veröffentlichungstag und Bekanntmachung des Hinweises auf die Patenterteilung: **25.09.2002**
(21) Anmeldenummer: 97905075.4
(22) Anmeldetag: 22.02.1997
(51) Int. Cl.: A61F 13/02, A61B 10/00, G01N 31/22, G01N 33/52

(54) **SELBSTKLEBENDER INDIKATOR**
SELF-ADHESIVE INDICATOR
INDICATEUR AUTOCOLLANT

(30) Priorität: 02.03.1996 DE 19608129
(43) Veröffentlichungstag der Anmeldung: 16.12.1998
(73) Patentinhaber: Schaefer, Andreas, 40468 Düsseldorf (DE); Stoldt, Volker Dr., 42781 Haan (DE)
(72) Erfinder: SCHAEFER, Andreas, D-40468 Düsseldorf (DE); DECKEN, Klaus, D-40227 Düsseldorf (DE); SCHMIDT, Karl, H., D-41466 Neuss (DE); STOLDT, Volker, D-40699 Erkrath (DE)
(86) Internationale Anmeldenummer: EP9700855
(87) Internationale Veröffentlichungsnummer: WO97031599

(56) Entgegenhaltungen:
- GB-A- 971 584
- GB-A- 2 034 033

## Beschreibung

Die Erfindung betrifft einen selbstklebenden Indikator.

Aus der Praxis sind Indikatoren für die verschiedensten Zwecke bekannt. Die meisten handelsüblichen Indikatoren liegen als Lösung vor, die in die zu untersuchende Substanz hineingegeben oder dort aufgebracht werden kann. Ein Beispiel für einen weithin bekannten Indikator ist das Phenolphthalein, das bei Erreichen eines bestimmten pH-Werts einen Farbumschlag zeigt. Es sind außerdem Indikatoren bekannt, die aus einem Trägermaterial gefertigt sind, das mit einer entsprechenden Indikatorlösung getränkt ist. Diese Indikatoren werden zur Durchführung einer bestimmten Untersuchung in die in Lösung befindliche, zu untersuchende Substanz eingetaucht oder damit benetzt. Bekannte Ausführungsformen dieser Indikatoren sind das pH-Papier sowie Indikatorstäbchen zur Urinuntersuchung.

Die GB 971584 A zeigt einen aufklebbaren Indikator zur Feststellung von Feuchtigkeit in Materialien. Ein feuchtigkeitssensitiver Indikator ist in einer mit Klebeband verschlossenen Hülle angeordnet und kann mit diesem Band auf die zu prüfende Oberfläche aufgeklebt werden.

Die GB 2034033A zeigt einen Indikator auf Flüssigkristallbasis, bei dem die Indikatorsubstanz zur Anzeige von Temperaturänderungen zwischen einem transparenten Material und einem schwarzen Hintergrund angeordnet ist. Die Indikatorsubstanz ist durch die Hintergrundschicht von dem Klebstoff separiert.

Außerdem sind aus der DE 30 07 744 klebende Indikatorpasten bekannt, die, auf ein Trägermaterial aufgetragen, Nachweise ermöglichen. Derartige Indikatorpasten verlieren beim anschließenden Trocknungsvorgang ihre Klebeeigenschaften.

Weiterhin ist aus der DE 39 13 868 C1 ein selbstklebender Teststreifen bekannt, der die Reagenzien und/oder Indikatoren nicht im Klebstoff, sondern auf der nichtklebenden Folienrückseite vorhält.

Der Einsatz der bekannten Indikatoren zur Untersuchung von Substanzen ist im wesentlichen auf flüssige Substanzen beschränkt, die in handhabbarer Form, beispielsweise in Reagenzgläsern etc. vorliegen. Der Einsatz von Indikatoren unter weniger standardisierten Bedingungen ist kaum möglich. Insbesondere sind bislang keine Indikatoren bekannt, die auf die menschliche Haut oder auf sonstige Gegenstände vorübergehend oder dauerhaft aufgebracht werden können, um dort quasi in situ bestimmte Substanzen oder physikalisch-chemische Zustände anzuzeigen.

Es ist deshalb Aufgabe der Erfindung, einen selbstklebenden Indikator zu schaffen, der neuartige Anwendungsmöglichkeiten für Indikatoren eröffnet. Diese Aufgabe wird von einem selbstklebenden Indikator mit den Merkmalen des Anspruchs 1 gelöst.

Weil der Indikator einen dünnen, beispielsweise folienartigen oder blattartigen Träger, eine einseitig auf dem Träger angeordnete Klebeschicht, wenigstens eine Indikatorsubstanz, die der Klebeschicht zugeordnet ist, indem die Indikatorsubstanz im wesentlichen homogen in die Klebstoffschicht eingemischt ist und zumindest bereichsweise durch den Träger sichtbar ist, aufweist, kann der Indikator an eine weitgehend beliebige Stelle geklebt werden. Die der Klebeschicht zugeordnete Indikatorsubstanz kann in Wechselwirkung mit dem beklebten Substrat treten und in Abhängigkeit von den auf dem Substrat befindlichen Stoffen oder den aus dem Substrat austretenden Stoffen beispielsweise einen Farbumschlag anzeigen. Weil der Indikator durch den Träger sichtbar ist, besteht die Möglichkeit, den Zustand des Indikators von außen durch Betrachtung oder Messung zu bestimmen. Auf diese Weise werden zumindest qualitative Aussagen über das Vorhandensein gewisser Stoffe möglich, es sind aber auch quantitative Nachweismethoden denkbar.

Eine besonders einfache Bestimmung des Indikatorzustandes ist möglich, wenn der Indikatornachweis auf einer Änderung der optischen Eigenschaften der Indikatorsubstanz basiert. Es ist dabei vorteilhaft, wenn die optischen Eigenschaften des Trägers so gestaltet sind, daß die Indikatorsubstanz mikroskopisch durch das Trägermaterial hindurch untersuchbar bleibt. Eine einfache Auswertung des Indikatorzustandes und damit der gewünschten Information ist möglich, wenn der Träger eine auf den Änderungsbereich der optischen Eigenschaften der Indikatorsubstanz abgestimmte Vergleichsskala trägt. Durch einfachen Vergleich zwischen dem Zustand der Indikatorsubstanz und der auf dem Träger angebrachten Skala ist dann zumindest ein halbquantitativer Meßwert zu erhalten.

Der selbstklebende Indikator wird auch bei Indikatorsubstanzen, die gegen äußere Einflüsse empfindlich sind, auf Dauer haltbar, wenn die Indikatorsubstanz in dauerhafter, bei Bedarf freisetzbarer Form der Klebeschicht zugeordnet ist. Vorteilhaft ist die Einbringung der Indikatorsubstanz in Mikrokapseln, die zur Freisetzung der Indikatorsubstanz zerdrückt werden können. Wenn die Klebeschicht mehrere Bereiche mit wenigstens zwei verschiedenen Indikatorsubstanzen nebeneinander aufweist, ist der simultane Test auf verschiedene zu untersuchende Substanzen möglich. Der Träger kann vorteilhaft aus einer Kunststoffolie gefertigt sein, die als Band oder als Klebepad ausgeführt ist. Derartige Kunststoffolien können von großer mechanischer Stabilität und günstigen optischen Eigenschaften sein. In gewissen Fällen ist es aber auch vorteilhaft, daß der Träger aus einem Gewebe, beispielsweise einem Textil- oder Metallgewebe gefertigt ist. Das Gewebe kann aus Naturfasern gefertigt sein, wenn die Anwendung von Kunststoffen nicht erwünscht ist. Bei prinzipiell undurchsichtigen Materialien kann dann der mit der Indikatorsubstanz versetzte Klebefilm durch die Maschen des Gewebes erkennbar gemacht werden.

Je nach Trägermaterial ist der Träger bevorzugt durchsichtig oder mit Durchbrüchen versehen.

Die Klebeschicht kann zur Verbesserung der Handhabbarkeit im unbenutzten Zustand mit einer abziehbaren Schutzfolie versehen sein. Diese Schutzfolie kann sowohl gegenüber mechanischen Beeinträchtigungen der Klebeschicht als auch gegenüber vorzeitig in Gang gesetzten Reaktionen mit der Indikatorsubstanz schützen.

Vorteilhafte Anwendungsmöglichkeiten ergeben sich, wenn die Indikatorsubstanz zur Anzeige von pH-Werten, zum Nachweis von Metaboliten, zur Anzeige von Peptiden, insbesondere Proteinen oder zur Anzeige von Kohlenhydraten geeignet ist. Wenn die Indikatorsubstanz zur Anzeige von Glucose geeignet ist, ergeben sich vorteilhafte medizinische Anwendungsmöglichkeiten. Wenn die Indikatorsubstanz zur Anzeige von Lipiden geeignet ist, sind insbesondere kriminaltechnische und forensische Anwendungen bevorzugt möglich. Schließlich kann die Indikatorsubstanz zur Anzeige von Nucleinsäuren, zur Anzeige von Antigen-/Antikörperreaktionen, zur Anzeige von biologischem Wachstum oder zur Anzeige von toxischen Substanzen geeignet sein. Auch für diese Nachweisreaktionen ergeben sich Anwendungsmöglichkeiten, die gegenüber den bisherigen Indikatoren ausgesprochen vorteilhaft sind. Besonders spezifische oder besonders empfindliche Nachweise sind möglich, wenn die Indikatorsubstanz mehrere im Gebrauch miteinander reagierende Komponenten enthält. Dabei kann die Indikatorsubstanz zur Verbesserung der Dauerhaltbarkeit auch so gewählte werden, daß sie erst nach Freisetzung mit dem Luftsauerstoff oder der Luftfeuchtigkeit reagiert und dadurch aktiv wird.

Im folgenden werden Ausführungsbeispiele der vorliegenden Erfindung anhand der Zeichnungen sowie anhand von mehreren Beispielen erläutert. Es zeigen:
- Fig. 1: einen erfindungsgemäßen selbstklebenden Indikator in Ausführungsform als rechteckiges Pflaster;
- Fig. 2: einen erfindungsgemäßen selbstklebenden Indikator bei seiner Anwendung als Diagnosehilfe bei dermatologischen Untersuchungen auf dem Unterarm eines Patienten; sowie
- Fig. 3: einen erfindungsgemäßen Indikator in einer Ausführungsform zum Nachweis von aus Möbeln austretenden, gasförmigen Schadstoffen.

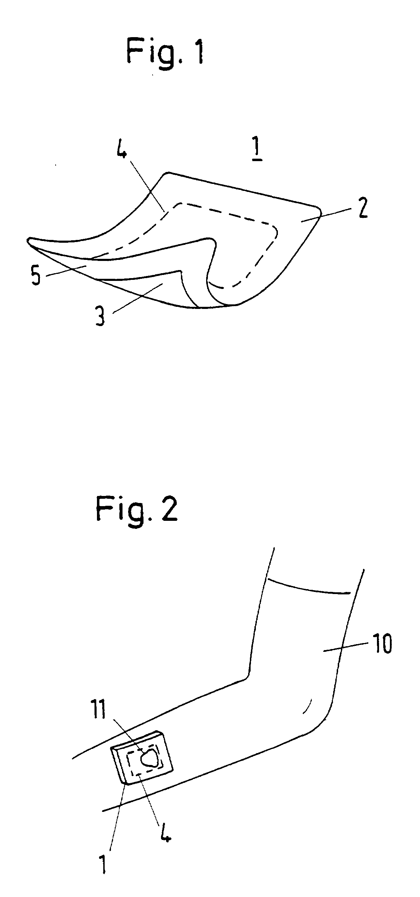

In der Figur 1 ist ein erfindungsgemäßer Indikator insgesamt mit 1 bezeichnet. Der Indikator 1 weist einen rechteckigen, folienartigen Träger 2 auf, der einseitig eine Klebeschicht 3 trägt. Die Klebeschicht 3 ist in der Figur 1 an der Unterseite des Trägers 2 angeordnet, während sie durch den transparenten Träger hindurch sichtbar ist, was in der Figur 1 mit 4 angedeutet ist. Zur Herstellung der Klebstoffschicht werden handelsübliche Haftklebstoffe auf Acrylsäureesterbasis, andere Haftklebstoffe, Wollwachse, Klebrigmacher und gegebenenfalls Hilfsstoffe bedarfsgemäß miteinander vermischt und auf einen Träger aufgebracht.

Die Klebeschicht 3 trägt die in der Figur nicht dargestellte Indikatorsubstanz. Um einen im aufgeklebten Zustand des Indikators 1 unbefestigten Rand zu vermeiden, ist außerhalb der Klebeschicht 3 ein Rand 5 des Trägers ebenfalls mit einer Klebeschicht versehen, die jedoch keine Indikatorsubstanz trägt.

In der Figur 2 ist stilisiert ein Arm 10 eines Patienten dargestellt, auf den im Bereich des Unterarms ein erfindungsgemäßer Indikator 1 aufgeklebt ist. Die von außen sichtbare Klebeschicht 3 steht mit der Haut des Patienten in Kontakt. Falls der Patient flächige Hauterkrankungen aufweist, kann die mit einem geeigneten Indikator für Stoffwechselprodukte der erkrankten Hautpartien oder für den pH-Wert der Hautpartien versehene Klebeschicht in einem bei 11 angedeuteten Bereich das Vorhandensein einer bestimmten Hautkrankheit, die flächenmäßige Ausdehnung dieser Hautkrankheit sowie sonstige Parameter der erkrankten Bereiche anzeigen. Verschiedene Hautkrankheiten, bei denen hinsichtlich der Ursachen und der Wirkungsweisen noch Aufklärungsbedarf herrscht, können mit den erfindungsgemäßen Indikatoren auf diese Weise einfach, zuverlässig und mit einer gewissen Ortsauflösung untersucht werden.

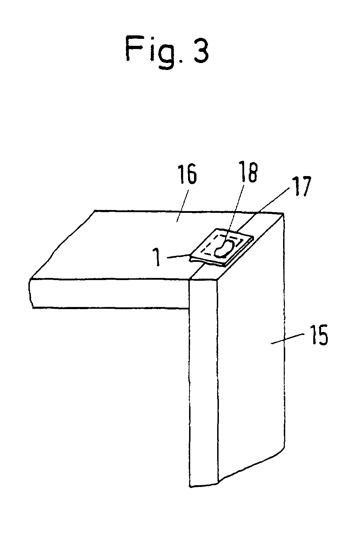

In der Figur 3 ist eine Ecke eines Möbelstücks dargestellt, das in abgebrochener Darstellung eine Seitenwand 15 und eine Deckenwand 16 aufweist. Die beiden Wände stoßen in einer Fuge 17 aneinander. Von außen ist ein selbstklebender Indikator auf die aneinander angrenzenden Oberflächen der Wände 15 und 16 aufgeklebt, und zwar derart, daß die Fuge 17 überdeckt wird. Wenn zur Herstellung des Möbelstücks Spanplatten verwendet wurden, die mit einer Oberflächenbeschichtung aus Kunststoff versehen sind, so treten verdampfende, also gasförmige Schadstoffe bevorzugt in den Bereichen aus den Materialien aus, die nicht mit einer Oberflächenversiegelung versehen sind, also an den Fugen. Beispielsweise sind in der Vergangenheit zur Herstellung von Möbelstücken Spanplatten unter Verwendung des Lösungsmittels Formaldehyd hergestellt worden, deren Emissionswerte die in Wohn- und Arbeitsräumen zulässigen Grenzwerte bei weitem überschritten haben. Ein solches Austreten von Formaldehyd aus der Fuge 17 zwischen den Wänden 15 und 16 des Möbelstücks kann durch eine Verfärbung des selbstklebenden Indikators bei 18 zumindest qualitativ nachgewiesen werden.

Im folgenden werden verschiedene Beispiele für Ausführungsformen des erfindungsgemäßen Indikators aufgeführt. Der erfindungsgemäße selbstklebende Indikator kann zur Umweltanalytik oder zur Diagnose von Hautkrankheiten mit einer Indikatorsubstanz ausgestattet werden, die pH-Werte anzuzeigen vermag. Hierzu sind aus der Chemie verschiedene Indikatoren bekannt, deren Farbumschlag innerhalb eines eng umrissenen pH-Bereichs liegt. So hat beispielsweise Bromphenolblau einen Farbumschlag bei pH = 4, Bromphenolrot bei pH = 6, Phenolrot bei pH = 8 und Phenolphthalein bei pH = 10. Verschiedene marktübliche Universalindikatoren sind auch zur Anzeige eines weiten pH-Bereichs verwendbar. So kann z. B. die Klebeschicht mit einer Indikatormischung nach Yamada versetzt werden, die im Bereich zwischen pH = 4 und pH = 10 verschiedene Farben annimmt. Zur optischen oder maschinellen Auswertung der Anzeige ist in diesem Fall der Aufdruck einer Vergleichsskala auf dem Trägermaterial des Indikators sinnvoll.

Bei Patienten, die an Diabetes mellitus erkrankt sind, steigt bei einer Insulinunterversorgung der Glucosegehalt des Blutes stark an. Die Messung des Glucosegehaltes des Blutes ist derzeit nur mit Methoden möglich, bei denen Blut aus dem Körper entnommen wird. Dieser Vorgang ist für den Patienten unangenehm, selbst, wenn es sich nur um die Gewinnung eines Blutstropfens aus dem Finger handelt. Hier können erfindungsgemäße selbstklebende Indikatoren eingesetzt werden, die in der Nähe von Blutgefäßen auf die Haut aufgeklebt werden und den klinisch relevanten Metaboliten Glucose über einen spezifischen enzymatischen Nachweis anzeigen. Beispielsweise ist ein probater spezifischer Nachweis mittels Glucose-Oxidase, Peroxidase und einem geeigneten Elektronenakzeptor für die Peroxidase möglich. Sobald der Glucosegehalt im Blut des Patienten zu hoch wird, kann der selbstklebende Indikator über die Nachweisreaktion einen Farbumschlag anzeigen, so daß der Patient eine Information darüber enthält, wann Insulin extern zugeführt werden muß.

Peptidische Verbindungen lassen sich direkt mittels der Biuret-Reaktion nachweisen, bei der Kupfer-II-Ionen zu Kupfer-I reduziert werden. Als Komplexbildner und zur Stabilisierung und Sichtbarmachung der Kupfer-I-Ionen ist heute die Bicinchonsäure bewährt. Gegebenenfalls ist eine Wärmebehandlung zum Erreichen einer ausreichenden Empfindlichkeit durchzuführen. Freie Aminogruppen von Peptiden und Proteinen, also entweder die endständigen α-Aminogruppen oder Aminogruppen in den Seitenketten von Diaminosäuren, wie Lysin, reagieren auch zu Schiff' sehen Basen und lassen sich so mit entsprechenden farbgebenden Reagenzien nachweisen, die eine Aldehydgruppe enthalten. Für besondere Subspezies von Peptiden bzw. Proteinen gibt es besondere Nachweisreaktionen:

Proteine mit einer Enzymaktivität lassen sich durch Ausnutzung dieser Aktivität nachweisen, indem sie zur Umsetzung eines chromogenen Substrates herangezogen werden. Beispielsweise kann Hämoglobin durch seine Peroxidase-Akivität Benzidin in Anwesenheit von Wasserstoffperoxid zu einem dunkelbraunen Produkt umsetzen. Auf diesem Wege lassen sich Blutspuren relativ sicher identifizieren.

Glycopeptide/Glycoproteine lassen sich mittels einer Schiff-Base-Reaktion nachweisen, gegebenenfalls nach einer Periodatoxidation. Das Schiff-Reagenz zum Nachweis von Aldehyden muß ein farbgebendes Reagenz mit einer Amino- oder Hydrazingruppe sein. Bewährt ist hier Fuchsin-schweflige Säure. Weiterhin läßt sich letztlich jedes Peptid oder Protein mittels durch Immunisierung gewonnene Antikörper spezifisch nachweisen. Derart ausgestattete selbstklebende Indikatoren sind als mikroskopischer Nachweis von Partikel wie z. P. Pilzhyphen und Spermien geeignet.

Lipide lassen sich durch Färbung mit einem fettlöslichen Farbstoff nachweisen, der in den Indikator eingebracht ist. Diese Farbstoffe können beispielsweise mit den Fettspuren von Fingerabdrücken spezifisch reagieren und dadurch nicht nur das Vorhandensein von Fingerabdrücken, sondern auch deren geometrische Form anzeigen. Als fettlösliche Farbstoffe kommen beispielsweise Sudanrot G oder Sudan III in Frage.

Zum mikroskopischen Nachweis von kohlehydrathaltigen Partikeln wie z. B. Stärke, Pollen, Pilzen und Blut ist der Nachweis von Kohlehydraten erwünscht. Kohlehydrate mit einer freien Aldehydgruppe lassen sich allgemein durch ein Reagenz auf Aldehydgruppen nachweisen. Bewährt sind beispielsweise Fehling'sches Reagenz, Schiff'sche Basenreaktion oder die Reaktion mit einem Hydrazin zu einem farbigen Hydrazon. Nötigenfalls können Kohlehydrate ohne freie Aldehyde durch eine limitierte Periodatoxidation aktiviert werden und anschließend mit einem Aldehydreagenz nachgewiesen werden. Bestimmte Kohlehydrate lassen sich spezifisch auch enzymatisch nachweisen. Beispielsweise ist ein bewährter spezifischer Nachweis von Glucose mittels Glucose-Oxidase möglich. Ein spezifischer Nachweis von Kohlehydraten ist auch mit Hilfe von sogenannten Lektinen möglich, die ähnlich wie Antikörper in hochspezifischer Weise mit definierten Kohlehydratverbindungen reagieren. Auf diesem Wege läßt sich beispielsweise selbst die Blutgruppe von Blutspuren feststellen, wenn die verwendeten Lektine mit einem Enzym wie z. B. Peroxidase gekoppelt sind. Es kann in solchen Fällen erforderlich sein, die zu untersuchenden Proben zunächst mit einem selbstklebenden Indikator 1 für eine erste Reaktionsstufe aufzunehmen, die mit einem Indikator reagierende Substanz dann mit einem zweiten Indikator abzunehmen, auf dem eine weitere Stufe der Nachweisreaktion erfolgt. Sodann wird ein dritter selbstklebender Indikator über die Reaktionszwischenprodukte geklebt, in dem dann letztlich die optisch wirksame Reaktion mit einem chromogenen Substrat erfolgt.

Nucleinsäuren lassen sich spezifisch durch solche Farbstoffe nachweisen, die zwischen den Basen der RNA bzw. den Basenpaaren der DNA interkalieren. Bewährt ist die Färbung mit Ethidiumbromid, das nach Bindung an Nucleinsäuren eine rote Fluoreszenz zeigt, oder Acridinorange, das mit RNA eine rote und mit DNA eine grüne Fluoreszenz zeigt. In diesem Fall ist sogar eine Unterscheidung zwischen RNA und DNA möglich. Für die Auswertung dieser Substanzen ist UV-Strahlung notwendig. Es stehen aber auch Farbstoffe als Indikatorsubstanz zur Verfügung, die auch bei sichtbarem Licht ausgewertet werden können. Diese Indikatorsubstanzen sind außerdem weniger toxisch als Ethidiumbromid oder Acridinorange.

Zur Untersuchung wird ein selbstklebender Indikator mit seiner Klebeschicht auf das zu untersuchende Material aufgebracht. Die der Klebeschicht zugeordneten Indikatorsubstanzen reagieren mit der Probe und können dann gegebenenfalls unter UV-Licht ausgewertet werden. Hierzu kann der selbstklebende Indikator von der untersuchten Substanz oder dem untersuchten Substrat abgezogen werden. Es ist aber auch möglich, den selbstklebenden Indikator an seinem Einsatzort zu belassen und dort auszuwerten.

Wenn Träger und Klebeschichten mit Brechungsindizes nahe dem des Glases verwendet werden, ist auch eine mikroskopische Auswertung der gefärbten Probe ohne Entfernen des Klebefilmes denkbar. Die optischen Eigenschaften sind dann so zu wählen, daß sie mit der Optik eines mikroskopischen Untersuchungsgeräts kompatibel sind.

Antigen/Antikörperreaktionen ermöglichen den Nachweis eines außerordentlich breiten Spektrums an Zielsubstanzen, erfordern jedoch derzeit noch eine mehrstufige Reaktion und legen damit eine technische Realisierung mit mehreren Indikatorschichten unterschiedlicher Zusammensetzung nahe. Ein erster selbstklebender Indikator enthält einen monoklonalen Antikörper oder ein polyklonales Antikörpergemisch, das gegen die Zielsubstanz gerichtet ist. Der erste selbstklebende Indikator wird auf die zu untersuchende Fläche aufgeklebt und nach einer angemessenen Reaktionszeit mit dort vorhandener Zielsubstanz wieder abgezogen und auf einen zweiten selbstklebenden Indikator aufgebracht. Der zweite selbstklebende Indikator enthält einen zweiten monoklonalen Antikörper oder auch polyklonale Antikörper gegen die Zielsubstanz. Dieser zweite Antikörper ist kovalent an ein Enzym gekoppelt, das im dritten Schritt die Farbreaktion bewerkstelligt. Dazu wird der erste selbstklebende Indikator nach einer angemessenen Reaktionszeit von dem zweiten selbstklebenden Indikator abgehoben und auf einen dritten selbstklebenden Indikator übertragen. Der dritte selbstklebende Indikator enthält schließlich ein Substrat für das Enzym. Im Falle der Peroxidase als Nachweisenzym ist beispielsweise Wasserstoffperoxid und Diaminobenzidin vorgesehen. Das Diaminobenzidin wird durch eine Peroxidaseeinwirkung zu einem dunkelbraunen Produkt umgesetzt. Andere farbgebende Substrate für die Peroxidase wie z. B. 4-Chlornaphthol, Tetramethylbenzidin, etc. oder andere Enzyme wie z. B. alkalische Phosphatase, β-Galaktosidase etc. mit ihren spezifischen Substraten 5-Chlor-4-brom-1-naphthylphosphat bzw. -β-D-galaktosid sind möglich.

Ein Vorteil dieser Anwendung liegt in der Möglichkeit, auch eine quantitative Auswertung der Färbung mittels eines Photometers vorzunehmen oder den selbstklebenden Indikator zur Dokumentation und zur semiquantitativen Auswertung auf ein weißes Papier aufzukleben.

Die Zielsubstanz, gegen die die Antikörper im ersten selbstklebenden Indikator gerichtet sind, können beispielsweise die Allergene der Hausstaubmilbe oder Pollenallergene sein. Einsetzbar wären auch Antikörper gegen Blutgruppenantigene, um so beispielsweise die Identität von Blutspuren festzustellen. Es ist auch der Nachweis von abiogenen Substanzen wie etwa Nitrophenol möglich.

Als ein Beispiel für umweltrelevante Chemikalien ist der Nachweis von Formaldehyd interessant. Die Indikatorsubstanz ist dabei nach einem Ausführungsbeispiel Fuchsin-schweflige Säure, die in die Klebeschicht eingebracht ist. Nach Einwirkung von Aldehyden ist ein Farbumschlag von farblos nach rot erfolgt. Wenn der spezifische Nachweis von Formaldehyd gewünscht ist, kann durch anschließend Aufbringen des selbstklebenden Indikators auf einen zweiten, salzsäurehaltigen Träger ein spezifischer Farbumschlag nach blau nachgewiesen werden.

Weitere Möglichkeiten eines Nachweises umweltrelevanter Chemikalien liegen in der Nachweisreaktion von Schwermetallionen mittels farbgebender Komplexbildner, die der Klebeschicht des selbstklebenden Indikators zugeordnet sind. So bilden etwa Quecksilber (-II)-Ionen mit Dithizon einen gelben Niederschlag oder Eisen(-II bzw. -III)-Ionen mit Hexacyanoferrat einen blauen Niederschlag, der auch als Berliner Blau bzw. Turnbulls Blau bekannt ist.

Ein Nachweis von mikrobieller Kontamination läßt sich durch die Einbringung eines spezifischen Nährmediums in die Klebeschicht des selbstklebenden Indikators bewerkstelligen. Die Kontaminanten können von den zu untersuchenden Flächen direkt mittels des selbstklebenden Indikators abgenommen werden, der danach inkubiert werden kann. Für medizinisch relevante Keime ist in der Regel eine Inkubation bei 37 °C erforderlich. Die Spezifität des Nachweises kann durch die Wahl eines Selektiv-Nährmediums erreicht werden, das entweder nur das Wachstum spezifischer Keime zuläßt oder nur bei Annwesenheit spezifischer Keime eine typische, optisch erkennbare Reaktion in der Indikatorsubstanz der Klebeschicht durchführt. Eine Vielzahl solcher Medien ist heute bekannt. So ist beispielsweise der Wasserblau-Metachromgelb-Lactose-Agar (Gassner-Nährboden) zum spezifischen Nachweis von Enterobakterien aus Lebensmitteln oder der Lysin-Eisen-Agar zum Nachweis von Salmonellen bekannt.

Biologisches Wachstum läßt sich mit erfindungsgemäßen selbstklebenden Indikatoren aber auch in anderer Form anzeigen. Dabei ist nach einem Ausführungsbeispiel die Klebeschicht mit einem Vitalfarbstoff versehen, der die Auswertbarkeit mit optischen Methoden sicherstellt. So ist beispielsweise das nur in lebenden Zellen zu gelbgrüner Fluoreszenz gebrachte Fluoreszeindiacetat oder das nur in toten Zellen rot fluoreszierende Trypanblau in die Klebeschicht einzuarbeiten. Als Alternative zu Trypanblau ist als Indikatorsubstanz Evans Blue möglich. Auch Kombinationen von verschiedenen Farbstoffen können in die Klebeschicht als Indikatorsubstanz eingebracht werden.

Auf diese Weise können hygienerelevante Keime in Kliniken und in der Lebensmittelhygiene untersucht werden. Es ist der Nachweis in Gestalt von Kulturen und als Direktnachweis möglich, je nach nachzuweisenden Kontaminanten und der gewählten Indikatorsubstanz.

## Patentansprüche

1. Selbstklebender Indikator mit einem dünnen, beispielsweise blattartigen oder folienartigen Träger,
mit einer einseitig auf dem Träger angeordneten Klebeschicht, sowie
mit wenigstens einer Indikatorsubstanz,
wobei die wenigstens eine Indikatorsubstanz der Klebstoffschicht zugeordnet ist, indem die Indikatorsubstanz im wesentlichen homogen in die Klebstoffschicht eingemischt ist, und wobei
die Indikatorsubstanz zumindest bereichsweise durch den Träger sichtbar ist.

2. Indikator nach Anspruch 1, **dadurch gekennzeichnet, daß** die Wirkung der Indikatorsubstanz auf einer Änderung der optischen Eigenschaften basiert.

3. Indikator nach einem der vorhergehenden Ansprüche, **dadurch gekennzeichnet, daß** der Träger derartige optische Eigenschaften aufweist, daß mikroskopische Untersuchungen durch den Träger hindurch möglich sind.

4. Indikator nach einem der vorhergehenden Ansprüche, **dadurch gekennzeichnet, daß** der Träger eine auf den Änderungsbereich der optischen Eigenschaften abgestimmte Vergleichsskala trägt.

5. Indikator nach einem der vorhergehenden Ansprüche, **dadurch gekennzeichnet, daß** die Indikatorsubstanz in dauerhaltbarer, bei Bedarf freisetzbarer Form der Klebeschicht zugeordnet ist.

6. Indikator nach einem der vorhergehenden Ansprüche, **dadurch gekennzeichnet, daß** die Klebeschicht mehrere Bereiche mit wenigstens zwei verschiedenen Indikatorsubstanzen nebeneinander aufweist.

7. Indikator nach einem der vorhergehenden Ansprüche, **dadurch gekennzeichnet, daß** der Träger aus einer Kunststoffolie gefertigt ist.

8. Indikator nach einem der vorhergehenden Ansprüche, **dadurch gekennzeichnet, daß** der Träger aus einem Gewebe gefertigt ist.

9. Indikator nach einem der vorhergehenden Ansprüche, **dadurch gekennzeichnet, daß** der Träger transparent ist.

10. Indikator nach einem der vorhergehenden Ansprüche, **dadurch gekennzeichnet, daß** der Träger Durchbrüche aufweist.

11. Indikator nach einem der vorhergehenden Ansprüche, **dadurch gekennzeichnet, daß** die Klebeschicht mit einer abziehbaren Schutzfolie versehen ist.

12. Indikator nach einem der vorhergehenden Ansprüche, **dadurch gekennzeichnet, daß**
die Indikatorsubstanz zur Anzeige von pH-Werten geeignet ist.

13. Indikator nach einem der vorhergehenden Ansprüche, **dadurch gekennzeichnet, daß**
die Indikatorsubstanz zum Nachweis von Metaboliten geeignet ist.

14. Indikator nach einem der vorhergehenden Ansprüche, **dadurch gekennzeichnet, daß** die Indikatorsubstanz zur Anzeige von Peptiden, insbesondere Proteinen geeignet ist.

15. Indikator nach einem der vorhergehenden Ansprüche, **dadurch gekennzeichnet, daß** die Indikatorsubstanz zur Anzeige von Kohlenhydraten und/oder Glucoseoxidase geeignet ist.

16. Indikator nach einem der vorhergehenden Ansprüche, **dadurch gekennzeichnet, daß** die Indikatorsubstanz zur Anzeige von Lipiden geeignet ist.

17. Indikator nach einem der vorhergehenden Ansprüche, **dadurch gekennzeichnet, daß** die Indikatorsubstanz zur Anzeige von Nucleinsäuren geeignet ist.

18. Indikator nach einem der vorhergehenden Ansprüche, **dadurch gekennzeichnet, daß** die Indikatorsubstanz zur Anzeige von Antigen/Antikörperreaktionen geeignet ist.

19. Indikator nach einem der vorhergehenden Ansprüche, **dadurch gekennzeichnet, daß** die Indikatorsubstanz zur Anzeige von biologischem Wachstum geeignet ist.

20. Indikator nach einem der vorhergehenden Ansprüche, **dadurch gekennzeichnet, daß** die Indikatorsubstanz zur Anzeige von toxischen Substanzen geeignet ist.

21. Indikator nach einem der vorhergehenden Ansprüche, **dadurch gekennzeichnet, daß** die Indikatorsubstanz zur Anzeige von Temperaturen und/oder Temperatureinwirkungen geeignet ist.

22. Indikator nach einem der vorhergehenden Ansprüche, **dadurch gekennzeichnet, daß** die Indikatorsubstanz mehrere im Gebrauch miteinander reagierende Komponenten enthält.

23. Indikator nach einem der vorhergehenden Ansprüche, **dadurch gekennzeichnet, daß** die Indikatorsubstanz durch Einwirkung von Luft, Sauerstoff und/oder Feuchtigkeit aktivierbar ist.

## Claims

1. Self-adhesive indicator with a thin, e.g. sheet-type or foil-type carrier with an adhesive layer applied to one side of the carrier, and with at least one indicator substance, whereby one (at least) indicator substance of the adhesive layer is allocated such that the indicator substance is mingled, largely homogeneously, in the adhesive layer, and whereby the indicator substance is visible through the carrier, at least in certain places.

2. Indicator conforming with claim 1, **characterised by** the fact that the influence of the indicator substance is based on a change in the optical properties.

3. Indicator conforming with one of the aforementioned claims, **characterised by** the fact that the carrier has optical properties such that microscopic investigations are possible through the carrier.

4. Indicator conforming with one of the aforementioned claims, **characterised by** the fact that the carrier has a comparison scale aligned to the spectrum of change of the optical properties.

5. Indicator conforming with one of the aforementioned claims, **characterised by** the fact that the indicator substance is allocated to the adhesive layer in a permanent form, and if need be, in a form that can be set free.

6. Indicator conforming with one of the aforementioned claims, **characterised by** the fact that the adhesive layer has several areas with at least two different, adjacent indicator substances.

7. Indicator conforming with one of the aforementioned claims, **characterised by** the fact that the carrier is made of plastic foil.

8. Indicator conforming with one of the aforementioned claims, **characterised by** the fact that the carrier is made of fabric.

9. Indicator conforming with one of the aforementioned claims, **characterised by** the fact that carrier is transparent.

10. Indicator conforming with one of the aforementioned claims, **characterised by** the fact that the carrier has gaps.

11. Indicator conforming with,one of the aforementioned claims, **characterised by** the fact that the adhesive layer has a protective foil that can be stripped off.

12. Indicator conforming with one of the aforementioned claims, **characterised by** the fact that the indicator substance is suitable for indicating pH values.

13. Indicator conforming with one of the aforementioned claims, **characterised by** the fact that the indicator substance is suitable for detecting metabolites.

14. Indicator conforming with one of the aforementioned claims, **characterised by** the fact that the indicator substance is suitable for indicating peptides, especially proteins.

15. Indicator conforming with one of the aforementioned claims, **characterised by** the fact that the indicator substance is suitable for indicating carbohydrates and/or glucose oxidase.

16. Indicator conforming with one of the aforementioned claims, **characterised by** the fact that the indicator substance is suitable for indicating lipids.

17. Indicator conforming with one of the aforementioned claims, **characterised by** the fact that the indicator substance is suitable for indicating nucleic acids.

18. Indicator conforming with one of the aforementioned claims, **characterised by** the fact that the indicator substance is suitable for indicating antigens/antibody reactions.

19. Indicator conforming with one of the aforementioned claims, **characterised by** the fact that the indicator substance is suitable for indicating biological growth.

20. Indicator conforming with one of the aforementioned claims, **characterised by** the fact that the indicator substance is suitable for indicating toxic substances.

21. Indicator conforming with one of the aforementioned claims, **characterised by** the fact that the indicator substance is suitable for indicating temperatures and/or temperature influence.

22. Indicator conforming with one of the aforementioned claims, **characterised by** the fact that the indicator substance contains several components that inter-react when in use.

23. Indicator conforming with one of the aforementioned claims, **characterised by** the fact that the indicator substance can be activated by the influence of air, oxygen and/or moisture.

## Revendications

1. Il s'agit d'un indicateur autocollant muni d'un substrat-porteur de faible épaisseur, par exemple du type feuille ou film, avec une couche adhésive disposée unilatéralement sur le substrat-porteur ainsi que d'au moins, une substance indicatrice qui, elle, doit être attribuée obligatoirement à l'enduction de colle, où l'essentiel est que la substance indicatrice doit être incorporée surtout de façon homogène dans la couche adhésive de la colle et que ladite substance indicatrice soit, au moins à différents endroits, visible à travers le substrat-porteur.

2. Indicateur d'après la revendication n°1, **caractérisé par le fait que** l'effet de la substance indicatrice repose sur un changement des propriétés optiques.

3. Indicateur d'après une des revendications précédentes, **caractérisé par le fait que** le substrat-porteur accuse des propriétés optiques du genre à permettre les examens microscopiques à travers le substrat-porteur.

4. Indicateur d'après une des revendications précédentes qui est **caractérisé par le fait que** le substrat-porteur porte une échelle de comparaison accordée avec le domaine modificatif.

5. Indicateur d'après une des revendications précédentes, **caractérisé par le fait que** la substance indicatrice est attribuée de façon durable et permanente à la couche adhésive et, si le cas se présente, avec la possibilité d'un dégagement éventuel.

6. Indicateur d'après une des revendications précédentes, **caractérisé par le fait que** la couche adhésive accuse plusieurs domaines avec au moins deux substances indicatrices juxtaposées.

7. Indicateur d'après une des revendications précédentes, **caractérisé par le fait que** le substrat-porteur est fabriqué à partir d'un film en matière plastique.

8. Indicateur d'après une des revendications précédentes, **caractérisé par le fait que** le substrat-porteur est fabriqué à partir d'un tissu.

9. Indicateur d'après une des revendications précédentes, **caractérisé par le fait que** le substrat-porteur est transparent.

10. Indicateur d'après une des revendications précédentes, **caractérisé par le fait que** le substrat-porteur accuse des percées.

11. Indicateur d'après une des revendications précédentes, **caractérisé par le fait que** la couche adhésive est munie d'un film protecteur pelable et détachable.

12. Indicateur d'après une des revendications précédentes, **caractérisé par le fait que** la substance indicatrice est appropriée à l'indication des indices pH.

13. Indicateur d'après une des revendications précédentes, **caractérisé par le fait que** la substance indicatrice est propre à déceler et à vérifier les métabolites.

14. Indicateur d'après une des revendications précédentes, **caractérisé par le fait que** la substance indicatrice est propre à afficher les peptides, notamment les protéines.

15. Indicateur d'après une des revendications précédentes, **caractérisé par le fait que** la substance indicatrice est propre à afficher les hydrates de carbone et/ou l'oxydoglucose

16. Indicateur d'après une des revendications précédentes, **caractérisé par le fait que** la substance indicatrice est propre à afficher les lipides.

17. Indicateur d'après une des revendications précédentes, **caractérisé par le fait que** la substance indicatrice est propre à afficher les acides nucléiques.

18. Indicateur d'après une des revendications précédentes, **caractérisé par le fait que** la substance indicatrice est propre à afficher les réactions de l'antigène.

19. Indicateur d'après une des revendications précédentes, **caractérisé par le fait que** la substance indicatrice est propre à afficher la croissance biologique.

20. Indicateur d'après une des revendications précédentes, **caractérisé par le fait que** la substance indicatrice est appropriée à afficher les substances toxiques.

21. Indicateur d'après une des revendications précédentes, **caractérisé par le fait que** la substance indicatrice est propre à afficher les températures et/ou les effets thermiques.

22. Indicateur d'après une des revendications précédentes, **caractérisé par le fait que** la substance indicatrice contient plusieurs composantes qui affichent une réaction interactive les unes avec les autres pendant l'usage.

23. Indicateur d'après une des revendications précédentes, **caractérisé par le fait que** la substance indicatrice peut être activée par l'action de l'air, de l'oxygène et/ou de l'humidité.
